# EUROPEAN PATENT SPECIFICATION

(11) **EP 2 091 526 B1**
(45) Date of publication and mention of the grant of the patent: **04.05.2011**
(21) Application number: 07858785.4
(22) Date of filing: 14.12.2007
(51) Int. Cl.: A61K 31/198, A61P 25/18, A61K 35/68

(54) **TREATMENT OF PERVASIVE DEVELOPMENTAL DISORDERS**
BEHANDLUNG PERVASIVER ENTWICKLUNGSSTÖRUNGEN
TRAITEMENT DE TROUBLES ENVAHISSANTS DU DÉVELOPPEMENT

(30) Priority: 14.12.2006 GB 0624879
(43) Date of publication of application: 26.08.2009
(73) Proprietor: SHS International Ltd., Liverpool L7 9PT (GB)
(72) Inventor: LYNCH, Andrew Sean, Merseyside L16 9JW (GB); HAMBLETON, Kerry Louisa, Cheshire CW9 8SB (GB); SMITH, Stephen Leyland, Merseyside L25 6JB (GB)
(74) Representative: Atkinson, Peter Birch
(86) International application number: PCT/GB2007/004815
(87) International publication number: WO 2008/071991

(56) References cited:
- WO-A-02/43507
- US-A1- 2004 005 304
- US-B1- 6 294 520

## Description

The present invention relates to the nutritional treatment of Pervasive Developmental Disorders, e.g. Autism.

### Background of the invention

Autism is a complex Developmental disease characterised by impairments in social functioning, communication and flexibility of thought and behaviour (Wing 96). Impairments become apparent in early childhood (-2yo) and last into adulthood, having a severe effect on an individual's learning and social integration.

Estimates of prevalence have increased markedly in recent years, probably due to improved diagnostic procedures and increased public and clinical awareness of the condition (Wing 02): current best estimates range from 1:1000 to 1:200 (Fombonne 99).

Autism is a member of an extended family of Pervasive Developmental Disorders (PDD), which also includes Asperger and Rett disorders. All PDDs require the triad of social, communication and behavioural impairments identified by Wing for diagnosis. Similar disorders, such as Attention Deficit Disorder (ADD), hyperactivity disorders and schizophrenia, generally exhibit such impairments, but their presence is not required for diagnosis. The group of disorders commonly presenting with Wing's triad, whether required for diagnosis or not, is often referred to as the 'autistic spectrum'.

Disease aetiology is unknown, with the consensus expert opinion being that a genetic predisposition may interact with multivariate environmental factors to cause the disorder. The uncertainty over possible causes and disease mechanisms has resulted in the poor therapeutic options available for affected individuals.

Current pharmaceutical treatments generally address secondary symptoms of the disease, not the core aspects. For example, neuroleptics are commonly used to moderate aggression and biting behaviour, SSRIs to treat depression, and stimulants to improve impulse control or hyperactivity. Such approaches are acknowledged to be of restricted worth in control of the condition generally (Bostic 05).

Opioid peptides in the urine of autistic children were first observed in the late 1980s (Israngkun 86). Such peptides are formed by processing of dietary gluten and casein proteins in the gut, and are normally not absorbed into the body. Gluten forms gliadomorphins and casein forms casomorphins. These peptides, collectively termed exorphins (Zioudrou 79), are of essentially identical structure and are potent psychosis-inducing factors (Lindstrom 84).

If gut structure is compromised, these peptides can enter the body. The 'leaky gut' or 'opioid excess' hypothesis was developed proposing that autism, at least in a subset of the population, may be caused by entry of exorphins into the body from a leaky gut, allowing transport in the blood to the brain, and subsequent damage to the brain.

The hypothesis has drawn support over the years from further characterisation of the urinary peptides (Shattock 90, Reichelt 91), definitive demonstrations of leaky gut in autistics (e.g., D'Eufemia 96), as well as structural characterisation of the GI pathology.

Food protein intolerance and/or food protein allergy have also been suggested to play a role in autism, and there appear to be parallel aspects between autism and inflammatory disorders. It has been hypothesised that the relationship between autism and GI immune dysregulation may be related to macroscopically observed Ileal Lymphoid hyperplasia and enterocolitis. Although Ileal lymphoid hyperplasia may be more prevalent in children with regressive Autism, it is also seen in children with food allergies and severe constipation, the latter being an extremely common finding in Autistic children.

Imbalances in immune and inflammatory processes in autistic patients have been reported. Peripheral Blood Mononuclear cells (PBMC's) obtained from Autistic children with GI symptoms produced more tumour necrosis factor ∝ (TNF ∝) than control subjects in response to stimulation with cow milk protein, suggesting a role of non IgE food allergy.

GI problems associated with autism have been noted and are estimated to affect approx. 20-30% of the population. Symptoms range from bloating and abdominal pain to loose stools and diarrhoea, and correlations with food intolerances, principally to milk and wheat, have been noted (e.g., Goodwin 71, Lightdale 01). Interestingly, urinal peptides are present in >90% of autistics, suggesting there may be a sub-clinical GI dysfunction in most autistics, which is only expressed as significant GI problems in the 20-30% noted above.

The original observations of urinary peptides and the leaky gut/opioid excess hypothesis led to the first clinical studies of dietary interventions, specifically casein- and gluten-free diets (Reichelt 90, Knivsberg 95, Lucarelli 95). Unfortunately, although these studies did suggest that dietary intervention could improve both GI and autistic measures over long time periods (up to 4 years), their design did not allow a direct effect between dietary control and clinical efficacy to be confirmed.

There are ongoing studies to confirm the efficacy of exclusion diet (e.g., NIMH 04), but one recent published study has achieved the methodological quality required to support clinical effect (Knivsberg 02). The study compared the impact of a casein/gluten-free diet versus normal diet on clinical autistic measures over 1 year in 20 autistic children with abnormally high urinary peptide levels. Significant reduction in autistic traits was observed in those on restricted diet. In addition to anecdotal reports from parents, case-studies and uncontrolled studies, this study lends 'tentative support' to dietary intervention as an effective option in autism (Millward 05).

The available data suggests that a sub-clinical GI impairment exists in the majority of autistics which is expressed as full-blown gastro intestinal symptoms such as decreased barrier function and diarrhoea, In a significant subset of the population Exclusion diets have been demonstrated to improve both GI and autistic function and to maintain the improvement for long time periods.

### Summary of the invention

According to a first aspect of the present invention there is provided the use of an orally administrable nutritional formulation comprising (a) free amino acids as the sole protein source, (b) fat and (c) carbohydrates wherein
a. the amino acid composition comprises at least L-alanine, L-arginine, L-aspartic acid, L-cystine, glycine, L-histidine, L-isoleucine, L-lysine, L-mathionine, L-phenytalanine, L-proline, L-serine, L-threonine, L-tryptophan, L-tyrosine, L-valine, L-carnitine and taurine;
b. the fat comprises long chain polyunsaturated fatty acids; and
c. the carbohydrates comprise dietary fibres
for the manufacture of a medicament for administration as the sole daily source of protein or as a nutritional supplement to exclusion diets, said amino acids in the nutritional formulation being the sole daily source of protein or supplementing in part the deficiencies caused by the exclusion of certain proteins in the diet for the treatment of Pervasive Developmental Disorders.

According to a second aspect of the present invention there is provided a nutritional formulation comprising (a) amino acids as the sole protein source, (b) fat and (c) carbohydrates wherein
a. the amino acid composition comprises at least L-alanine, L-arginine, L-aspartic acid, L-cystine, glycine, L-histidine, L-isoleucine, L-lysine, L-methionine, L-phenylalanine, L-proline, L-serine, L-threonine, L-tryptophan, L-tyrosine, L-valine, L-carnitine and taurine;
b. the fat comprises long chain polyunsaturated fatty acids; and
c. the carbohydrates comprise dietary fibres
for the treatment of Pervasive Developmental Disorders.

According to a third aspect of the present invention there is provided a composition comprising amino acids as the sole protein source, fat and carbohydrates wherein
a. the amino acid composition comprises at least L-alanine, L-arginine, L-aspartic acid, L-cystine, glycine, L-histidine, L-isoleucine, L-lysine, L-methionine, L-phenylalanine, L-proline, L-serine, L-threonine, L-tryptophan, L-tyrosine, L-valine, L-carnitine and taurine;
b. the fat comprises at least 10 wt% long chain polyunsaturated fatty acids based on the weight of the fat in the composition;
c. the carbohydrates comprising between 5 and 50 wt% dietary fibres based on the total weight of the carbohydrates in the composition; and
wherein the weight percentage of amino acids is at least 5%, of fat is up to 20% and the carbohydrates at between 10 and 70% of the dry weight of the composition.

### Detailed description of the invention

The inventors have found, and this forms the basis of the present invention, that oral administration of a nutritional formulation containing free amino acids as the sole protein source in the formulation (so that the nutritional formulation is devoid of protein and/or protein fragments) in conjunction with the nutritional Formulation providing the only protein source that is administered to the patient is an effective method for the treatment of Pervasive Developmental Disorders, Thus the patient receives no whole protein or protein fragments and there is therefore no risk of the production of exorphins such as gliadomorphins and casomorphins.

Additionally the inventors found that the compositions according to the invention can preferably be used as a supplemental formulation in order to overcome deficiencies caused by restrictions introduced in the diets of the patients and to improve their gastro intestinal health by improving the barrier function. In such a case the amino acid-based formulation is then included in a protein restricted diet of the PDD patients.

The nutritional formulation may be administered as the sole source of protein (i.e. as provided by the amino acids) for a limited period of time before dietary protein is reintroduced. This limited period of time may, for example, be at least one week, more generally at least two weeks. Typically the period would be 2-4 weeks or even longer depending on the circumstances. Reintroduction of dietary protein may follow an algorithm designed to detect which protein(s) is/are involved in individual patients.

In a preferred embodiment, the nutritional formulation may be administered as a supplement to the diet of these patients for an extended period of time. When the patients are advised to limit their normal protein intake this limitation can be compensated by an amino acid-based formulation according to this invention.

The free amino acids present in the formulation may comprise L-alanine, L-arginine, L-aspartic acid, L-cystine, glycine, L-histidine, L-isoleucine, L-lysine, L-methionine, L- phenylalanine, L-proline, L-serine, L-threonine, L-tryptophan, L-tyrosine, L-valine, L-carnitine and taurine. The nutritional formulation may further comprise L-glutamine as a free amino acid. The presence or absence of glutamine will generally be dictated by the manner in which the nutritional formulation is produced. If heat treatment is required (e.g. for the production of a nutritional formulation in the form of a pasteurised drink) then the use of glutamine will generally be avoided to prevent "off-flavours".

The formulation comprises in addition to the amino-acid based protein equivalent, a fat source comprising Long Chain Poly Unsaturated Fatty Acids (LC-PUFA) and dietary fibres, and optionally higher levels of micronutrients. The inventors believe that the combination of amino acids and LC-PUFA improves the gastro-intestinal (Gl) symptoms in patients suffering from Pervasive Developmental Disorders. A preferred embodiment comprises EPA and/or DHA as LC-PUFA. The preferred ratio omega-3/omega-6 Fatty acids are between 0.1 and 1, since within this ratio optimal effects are envisaged on both the intestinal symptoms and autistic traits.

Dietary fibres are used to improve the effects of amino acid formulations on intestinal barrier function of these patients. By careful selection of the ingredients the compositions will improve the gastro-intestinal symptoms and reduce autistic traits.

Dietary fibres as used in this invention are typically resistant to digestion and absorption in the human small intestine with preferably a complete or partial fermentation in the large intestine. Preferably the present competition comprises at least one dietary fibre selected from the group consisting of galactooligosaccharides including trans galactooligosaccharides, long chain FOS (inulin), short chain F0S (fructooligosaccharides), xylooligosaccharides, palatinoseoligosaccharide, soybean oligosaccharide, gentiooligosaccharide, pectin, pectate, alginate, chondroitine, hyaluronic acids, heparine, heparane, sialoglycans, fucoidan, fucooligosaccharides, carrageenan, xanthan gum, cellulose, polydextrose (PDX, a non-digestible carbohydrate that has been synthesized from randomly cross-linked glucose and sorbitol), guar gum, arabinoxylan preferably MGN-3 Rice Bran Arabinoxylan Compound according to US Patent: 5,560,914, xyloglycan, callose, lignin and/or degradation products of dietary fibres.

All of the here mentioned fibres have beneficial prebiotic effects in the intestinal system. It is estimated that total fibre intakes range between 15-25g/d for adults. Intake of 12.7-14.4 g per day have been estimated in children aged 4-7, rising to 17.3-21 Ig per day in 15-19 year olds, Intakes of Non Starch Polysaccharide has been suggested at 12-16g per day with a 40-50% contribution from soluble fibre and 50-60% from insoluble fibre. Intakes of Inulin and FOS range from 2-12g per day. Recommendations made at a US conference on dietary fibre in childhood for children over 2 years was an intake with a range of one grams for each year of age plus 5g to one grams of fibre for each year of age plus 10g⁵¹, Therefore a two year old would require 2-12g fibre per day.

Preferably, due to issues with gastrointestinal problems such as diarrhoea in autism, the composition according to the invention does not include insoluble fibre. Therefore, in a preferred embodiment the long chain FOS source Inulin raftiline (BeneoHP) will be provided in a product in combination with raftilose (BeneoP95) as a source of short chain FOS which are highly soluble dietary fibres.

In a further preferred embodiment probiotics are included in the composition according to the invention.

### Probiotic micro-organism

Probiotic micro-organism means a micro-organism which beneficially affects a patient with Pervasive Developmental Disorders, by improving its intestinal microbial balance. The probiotic micro-organism may be selected from one or more micro-organisms suitable for human consumption and which is able to improve the microbial balance in the intestine. Preferably, the present composition contains 10⁴ to 10¹², more preferably from 10⁵ to 10¹¹, most preferably from 10⁷ to 5x10¹⁰ colony forming units (cfu) of probiotic bacteria per gram uronic acid oligosaccharide with a DP between 2 and 100. The present composition preferably contains 10² to 10¹³ colony forming units (cfu) of probiotic bacteria per gram dry weight of the present composition, preferably 10⁴ to 10¹², more preferably 10⁵ to 10¹⁰, most preferably from 10⁵ to 1x10⁹ cfu. The dosage of probiotic bacteria according to the present invention is preferably between 10² to 10¹³, more preferably from 10⁵ to 10¹¹, most preferably from 10⁸ to 5x10¹⁰ colony forming units (cfu) per day. Preferably live or viable bacteria are used, but dead bacteria or bacterial fragments may also be used.

Preferably the present composition comprises bacteria of the genus Lactobacillus and/or Bifidobacterium. Preferably the composition comprises a Bifidobacterium selected from the group consisting of *B. longum, B.breve* and *B*. *bifidum* and/or a Lactobacillus selected from the group consisting of *L*. *casei, L paracasei, L. rhamnosus, L. acidophilus* and *L. plantarum.* Most preferably the present composition comprises *Bifidobacterium breve* and/or *Lactobacillus paracasei.*

*Bifidobacterium breve* is a Gram-positive, anaerobic, rod-shaped bacterium. The present *B*. *breve* preferably has at least 95 % nucleic acid sequence identity of the 16 S rRNA sequence when compared to the type strain of *B. breve* ATCC 15700, more preferably at least 97%, 98%, 99% or more sequence identity as defined in Stackebrandt & Goebel, 1994, Int. J. Syst. Bacteriol. 44:846-849. Nucleic acid sequence identity is calculated for two nucleotide sequences, when optimally aligned, using the programs GAP or BESTFIT using default parameters. The GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna (Henikoff & Henikoff, 1992, PNAS 89, 915-919). It is clear than when RNA sequences are said to be essentially similar or have a certain degree of sequence identity with DNA sequences, thymine (T) in the DNA sequence is considered equal to uracil (U) in the RNA sequence. Sequence alignments and scores for percentage sequence identity may be determined using computer programs, such as the GCG Wisconsin Package, Version 10.3, available from Accelrys Inc., 9685 Scranton Road, San Diego, CA 92121-3752, USA or EMBOSSwin v. 2.10.0. The *Bifidobacterium* used in the present invention preferably hybridises with the *B. breve* probe and gives a signal with the 5' nuclease assay method as described in WO 2005/039319 and WO 2006/091103, both in the name of N.V. Nutricia. According to a preferred embodiment, the present composition contains at least one *B. breve* selected from the group consisting of *B. breve* Bb-03 (Rhodia), *B. breve* M16-V (Morinaga), *B. breve* R0070 (Institute Rosell, Lallemand), DSM 20091, and LMG 11613. Most preferably, the *B*. *breve* is *B. breve* M-16V (Morinaga).

In a preferred embodiment the present composition comprises *Lactobacillus paracasei.* Preferably the present *L. paracasei* strain has at least 95%, more preferably at least 97%, 98%, 99% or more nucleic acid sequence identity of the 16S rRNA sequence when compared to the type strain of *L. paracasei* ATCC 25032 as defined above. The *Lactobacillus* used in the present invention preferably hybridises with the *L. paracasei* probe and gives a signal with the 5' nuclease assay method as described in co-pending european patent application 05075486.0 of the present applicant. According to a preferred embodiment, the present composition contains at least a *L*. *paracasei* selected from the group consisting of *L*. *paracasei* F19 (Arla, Sweden), *L. paracasei* LAFTI L26 (DSM Food Specialties, the Netherlands) and *L. paracasei* CRL 431 (Chr. Hansen, Denmark), LMG 12165 and LMG 11407.

In a preferred embodiment, the composition according to the invention comprises a particular strain, Lactobacillus casei DN- 114 001 (CNCM 1-1518), which is present in Actimel(R), for delaying and/or alleviating the symptoms of a hypersensitivity reactions that may be present in patients suffering from Pervasive Developmental Disorders, e.g. Autism.

In a further preferred embodiment the probiotic bacteria are combined with prebiotic bacteria. This combination called symbiotics are particularly useful for the treatment of intestinal symptoms in patients suffering from PDD.

Many micronutrients are found to be inefficiently absorbed in instances of GI dysfunction. Accordingly, key micronutrients can be provided at higher levels (i.e. greater than 100% of their current Dietary Reference Intake (DRI) values) in the invention to compensate, e.g., Vitamins A, B6, B12, C, folate, Mg, Zn, Ca. The following Table lists preferred ranges and amounts of these micronutrients (relative to their current DRI values) that are administered in accordance with the invention to a patient suffering from PDD.

| **Micronutrient** | **Preferred Range** | **Preferred Amount** |
|---|---|---|
| ¹Vit A | >100%-300% | 190% |
| ²Vit B6 | >100%-300% | 150% |
| ²Vit B12 | >100%-300% | 160% |
| ³Vit C | 300%-600% | 470% |
| ²Folate | >100%-300% | 150% |
| ¹Mg | 500%-750% | 680% |
| ⁴Zn | >100%-300% | 240% |
| ¹Ca | >100%-300% | 170% |

| | | |
|---|---|---|
| ¹ Dietary Reference Intakes for Calcium, Phosphorus, Magnesium, Vitamin D and Fluoride (1997) ² Dietary Reference Intakes for Thimanin, Riboflavin, Niacin, Vitamin B6, Folate,³ Vitamin B12, Pantothenic Acid, Biotin and Choline (1998) ³ Dietary Reference Intakes for Vitamin C, Vitamin E, Selenium and Carotenoids . (2000) ⁴ Dietary Reference Intakes for Vitamin A, Vitamin K, Arsenic, Boron, Chromium, Copper, Iodine, Iron, Manganese, Molybdenum, Nickel, Silicon, Vanadium and Zinc (2001) | | |

All values in the middle and right hand columns are with respect to the DRI values in the following reports of the Food and Nutrition Board, Institute of Medicine (all reports published by National Academy Press, Washington DC):

The formulation may be nutritionally complete.

### Use of the invention:

The invention is applicable particularly to the treatment of Autism but also has applicability to other Pervasive Developmental Disorders such as Asperger and Rett Disorders.

The compositions according to the invention can also be used for the treatment of intestinal disorders present in patients with pervasive developmental disorders. The compositions are in particular suitable for the treatment and/or prevention of intestinal barrier function and diarrhoea in this patient group.

Although the invention is intended primarily for the treatment of Pervasive Developmental Disorders, we also envisage that it has application to the diagnosis of such conditions. Thus the nutritional formulation may be administered to a person suspected of suffering from a Pervasive Developmental Disorder to see whether there is any improvement in the condition. If so, this would at least assist in confirming a diagnosis of the condition in that person.

The invention is illustrated by the following non-limiting Examples.

### Examples

Particularly suitable formulations for administration in accordance with the invention are Neocate, Neocate LCP, and Paediatric E028 liquid, all available from SHS International Limited.

Preferred embodiment for the treatment of pervasive developmental disorders per 100 gram liquid product.
- Amino acid protein equivalent 2.5
- Carbohydrate 14.6
- Fibre 5.2
   o FOS, FPS 2.6, 2.6 gram respectively
- Fat 3.5
   o MCT 1.5
   o LCT 2.0
- micronutrients in higher levels than the Dietary Reference Intake
- Water for up to 100 gram product

Preferred embodiment for the treatment of Autism (supplement) weight percent based on dry weight of the product
- Amino acid protein equivalent 13
- Carbohydrate 49
- Fat 23
   o MCT 7.6
   o LCT 15.4
- Dietary fibres 5
   o Short chain FOS, long chain FOS (inulin) 2.5, 2.5
- Vitamins and minerals
- Optionally probiotic bacteria may be present. 1

A particular formulation for use in accordance with the invention is a liquid having the composition set out in the following Table in which the amounts of the various components are expressed per 100 ml of liquid (the balance of the composition being water).

| **Nutrient Name** | | **Level per 100ml** |
|---|---|---|
| Protein Equivalent | | 2.5 g |
| Energy | | 100 kcals |
| Carbohydrate | | 14.6 g |
| Fat | (Total) | 3.5 g |
| | (MCT) | 35% |
| | (LCT) | 65% |
| | | |
| **Minerals** | | |
| Sodium | | 65 mg |
| Potassium | | 180 mg |
| Chloride | | 75 mg |
| Calcium | | 140 mg |
| Phosphorus | | 74 mg |
| Magnesium | | 25 mg |
| | | |
| **Trace Elements** | | |
| Iron | | 1.6 mg |
| Zinc | | 1.0 mg |
| Iodine | | 19.0 µg |
| Manganese | | 0.14 mg |
| Copper | | 0.12 mg |
| Molybdenum | | 4.8 µg |
| Selenium | | 3.2 µg |
| Chromium | | 4.0 µg |
| | | |
| **Vitamins** | | |
| Vitamin A | | 80 µg |
| Vitamin E | | 1.2 IU |
| L-Ascorbic Acid | | 10 mg |
| Thiamin | | 0.11 mg |
| Riboflavin | | 0.11 mg |
| Pyridoxine | | 0.11 mg |
| Niacin | | 0.95 mg |
| Niacin Equivalent | | 2 mg |
| Pantothenic Acid | | 0.42 mg |
| Myo-Inositol | | 23 mg |
| Choline | | 31 mg |
| Vitamin D3 | | 1.0 µg |
| Cyanocobalamin | | 0.2 µg |
| Folacin | | 32 µg |
| d-Biotin | | 2 µg |
| Vitamin K1 | | 4.3 µg |
| | | |
| **Amino Acids** | | |
| L-Alanine | | 0.11 g |
| L-Arginine | | 0.5 g |
| L-Aspartic Acid | | 0.2 g |
| L-Cystine | | 0.01 g |
| Glycine | | 0.18 g |
| L-Histidine | | 0.13 g |
| L-Isoleucine | | 0.18 g |
| L-Leucine | | 0.3 g |
| L-Lysine | | 0.22 g |
| L-Methionine | | 0.14 g |
| L-Proline | | 0.21 g |
| L-Phenylalanine | | 0.25 g |
| L-Serine | | 0.13 g |
| L-Threonine | | 0.15 g |
| L-Tryptophan | | 0.06 g |
| L-Tyrosine | | 0.05 g |
| L-Valine | | 0.19 g |
| L-Carnitine | | 3.17 mg |
| Taurine | | 6.62 mg |
| **Total Amino Acids** | | **3 g** |

### References

Bostic 2005, Expert Opin Emerg Drugs 10: 521.
D'Eufemia 1996, Acta Paediatr. 85:1076
Fombonne 1999, Psychol. Med. 29:769
Goodwin 1971, J. Autism Child Schiz.1:48
Harapocos 1975, DIPAB Heming, Norway
Israngkun 1986, Neurochem. Pathol. 5:51
Knivsberg 1995, Scand. J. Educ. Res. 39:223
Knivsberg 2002, Nutr. Neurosci. 5:251
Liacouras 2003, Am. J. Gastroenterol. 98:777
Lightdale 2001, Clin. Perspect. Gastroenterol. 1:56
Lindstrom 1984, Am. J. Psych. 41:1059
Lucarelli 1995, Panminerva Med. 37:137
Millward 2005, Cochrane Database Syst. Rev. CD003498
NIMH 2004, http:llclinicaltrials.gov/show/NCT00090428
Rainford 2005, Internal SHS report
Reichelt 1990, J. Appl. Nutr. 42:1
Reichelt 1991, Brain Dysfunct. 4:308
Shattock 1990, Brain Dysfunct. 3:328
Wing 1996, BMJ 312:327
Wing 2002, Mental Retardation Dev. Disabilities Res. Dev. 8:151
Zioudrou 1979, J. Biol. Chem. 254:2446

## Claims

1. Use of an orally administrable nutritional formulation comprising (a) free amino acids as the sole protein source, (b) fat and (c) carbohydrates wherein
a. the amino acid composition comprises at least L-alanine, L-arginine, L-aspartic acid, L-cystine, glycine. L-histidine, L-isoleucine, L-lysine, L-methionine, L-phenylalanine, L-proline, L-serine. L-threonine, L-tryptophan, L-tyrosine, L-valine, L-carnitine and taurine;
b. the fat comprises long chain polyunsaturated fatty acids;and
c. the carbohydrates comprise dietary fibres
for the manufacture of a medicament for administration as the sole daily source of protein or as a nutritional supplement to exclusion diets, said amino acids in the nutritional formulation being the sole daily source of protein or supplementing in part the deficiencies caused by the exclusion of certain proteins in the diet for the treatment of Pervasive Developmental Disorders.

2. Use as claimed in claim 1 wherein the Pervasive Developmental Disorder is autism.

3. The use as claimed in claim 1 wherein the Pervasive Developmental Disorder is Asperger disorder or Retts disorder.

4. Use as claimed in claim 1 wherein
b. the fat comprises at least 10 wt% long chain polyunsaturated fatty acids based on the weight of the fat in the composition;
c. the carbohydrates comprising between 5 and 50 wt% dietary fibres based on the total weight of the carbohydrates in the composition; and
wherein the weight percentage of amino acids is at least 5%, of fat up to 20% and the carbohydrates at between 10 and 70% of the dry weight of the composition.

5. The use as claimed in claim 4 wherein the free amino acids additionally comprise L-glutamine.

6. The use as claimed in any one of claims 1 to 5 wherein the formulation incorporates probiotic bacteria.

7. Nutritional formulation comprising (a) amino acids as the sole protein source, (b) fat and (c) carbohydrates wherein
a. the amino acid composition comprises at least L-alanine, L-arginine, L-aspartic acid, L-cystine, glycine, L-histidine, L-isoleucine, L-lysine, L-methlonine, L-phenylalanine, L-proline, L-serine, L-threonine, L-tryptophan, L-tyrosine, L-valine, b-carnitine and taurine;
b. the fat comprises long chain polyunsaturated fatty acids; and
c. the carbohydrates comprise dietary fibres
for the treatment of Pervasive Developmental Disorders.

8. A formulation as claimed in claim 7 wherein the Pervasive Developmental Disorder is autism.

9. A formulation as claimed in claim 7 wherein the Pervasive Developmental Disorder is Asperger disorder or Retts disorder.

10. A formulation as claimed in claim 7 wherein
b. the fat comprises at least 10 wt% long chain polyunsaturated fatty acids based on the weight of the fat in the composition;
c. the carbohydrates comprising between 5 and 50 wt% dietary fibres based on the total weight of the carbohydrates in the composition; and
wherein the weight percentage of amino acids is at least 6%, of fat up to 20% and the carbohydrates at between 10 and 70% of the dry weight of the composition.

11. A formulation as claimed in claim 10 wherein the free amino acids additionally comprise L-glutamine.

12. A formulation as claimed in any one of claims 7 to 11 wherein the formulation incorporates probiotic bacteria.

13. Composition comprising amino acids as the sole protein source, fat and carbohydrates wherein
b. the amino acid composition comprises at least L-alanine, L-arginine, L-aspartic acid, L-cystine, glycine, L-histidine, L-isoleucine, L-lysine, L-methionine, L-phenylalanine, L-proline, L-serine, L-threonine, L-tryptophan, L-tyrosine, L-valine. L-carnitine and taurine;
c. the fat comprises at least 10 wt% long chain polyunsaturated fatty acids based on the weight of the fat in the composition;
d. the carbohydrates comprising between 5 and 50 wt% dietary fibres based on the total weight of the carbohydrates in the composition; and
wherein the weight percentage of amino acids is at least 5%, of fat is up to 20% and the carbohydrates at between 10 and 70% of the dry weight of the composition.

14. A composition according to claim 13 further comprising probiotic bacteria.

## Patentansprüche

1. Verwendung einer oral verabreichbaren Ernährungsformulierung umfassend (a) freie Aminosäuren als einzige Proteinquelle, (b) Fett und (c) Kohlehydrate, wobei
a. die Aminosäurezusammensetzung mindestens L-Alanin, L-Arginin, L-Asparaginsäure, L-Cystin, Glycin, L-Histidin, L-Isoleucin, L-Lysin, L-Methionin, L-Phenylalanin, L-Prolin, L-Serin, L-Threonin, L-Tryptophan, L-Tyrosin, L-Valin, L-Carnitin und Taurin umfasst;
b. das Fett langkettige mehrfach ungesättigte Fettsäuren umfasst; und
c. die Kohlehydrate Ballaststoffe umfassen
für die Herstellung eines Medikaments zur Verabreichung als einzige tägliche Quelle von Protein oder als Nahrungsmittelergänzung bei Ausschlussdiäten, wobei die Aminosäuren in der Ernähnangsformulierung die einzige tägliche Quelle von Protein sind oder teilweise die Mängel ersetzen, die durch den Ausschluss gewisser Proteine aus der Nahrung zur Behandlung pervasiver Entwicklungsstörungen verursacht werden.

2. Verwendung nach Anspruch 1, wobei die pervasive Entwicklungsstörung der Autismus ist.

3. Verwendung nach Anspruch 1, wobei die pervasive Entwicklungsstörung das Asparger-Syndrom oder das Rett-Syndrom ist.

4. Verwendung nach Anspruch 1, wobei
b. das Fett mindestens 10 Ges.-% langkettige mehrfach ungesättigte Fettsäuren, auf das Gewicht des Fetts in der Zusammensetzung bezogen, umfasst;
c. die Kohlehydrate 5 bis 50 Gew.-% Ballaststoffe, auf das Gesamtgewicht der Kohlehydrate in der Zusammensetzung bezogen, umfassen; und
wobei der Gewichtsprozentsatz an Aminosäuren mindestens 5 %, an Fett bis zu 20 % und an Kohlehydraten 10 bis 70 %, auf das Trockengewicht der Zusammensetzung bezogen, beträgt.

5. Verwendung nach Anspruch 4, wobei die freien Aminosäuren zusätzlich L-Glutamin umfassen.

6. Verwendung nach einem der Ansprüche 1 bis 5, wobei in die Formulierung probiotische Bakterien eingearbeitet sind.

7. Emährungsformulierung umfassend (a) Aminosäuren als einzige Proteinquelle, (b) Fett und (c) Kohlehydrate, wobei
a. die Aminosäurezusammensetzung mindestens L-Alanin, L-Arginin, L-Asparaginsäure, L-Cystin, Glycin, L-Histidin, L-Isoleucin, L-Lysin, L-Methionin, L-Phenylalanin, L-Prolin, L-Serin, L-Threonin, L-Tryptophan, L-Tyrosin, L-Valin, L-Carnitin und Taurin umfasst;
b. das Fett langkettige mehrfach ungesättigte Fettsäuren umfasst; und
c. die Kohlehydrate Ballaststoffe umfassen
für die Behandlung von pervasiven Entwicklungsstörungen.

8. Formulierung nach Anspruch 7, wobei die pervasive Entwicklungsstörung der Autismus ist.

9. Formulierung nach Anspruch 7, wobei die pervasive Entwicklungsstörung das Asperger-Syndrom oder das Rett-Syndrom ist.

10. Formulierung nach Anspruch 7, wobei
b. das Fett mindestens 10 Gew.-% langkettige mehrfach ungesättigte Fettsäuren, auf das Gewicht des Fetts in der Zusammensetzung bezogen, umfasst;
c. die Kohlehydrate 5 bis 50 Gew.% Ballaststoffe, auf das Gesamtgewicht der Kohlehydrate in der Zusammensetzung bezogen, umfassen; und
wobei der Gewichtsprozentsatz an Aminosäuren mindestens 5 %, an Fett bis zu 20 % und an Kohlehydraten 10 bis 70 %, auf das Trockengewicht der Zusammensetzung bezogen, beträgt.

11. Formulierung nach Anspruch 10, wobei die freien Aminosäuren zusätzlich L-Glutamin umfassen.

12. Formulierung nach einem der Ansprüche 7 bis 11, wobei in die Formulierung probiotische Bakterien eingearbeitet sind.

13. Zusammensetzung umfassend Aminosäuren als einzige Proteinquelle, Fett und Kohlehydrate, wobei
b. die Aminosäurezusammensetzung mindestens L-Alanin, L-Arginin, L-Asparaginsäure, L-Cystin, Glycin, L-Histidin, L-Isoleucin, L-Lysin, L-Methionin, L-Phenylalanin, L-Prolin, L-Serin, L-Threonin, L-Tryptophan, L-Tyrosin, L-Valin, L-Carnitin und Taurin umfasst;
c. das Fett mindestens 10 Gew.-% langkettige mehrfach ungesättigte Fettsäuren, auf das Gewicht des Fetts in der Zusammensetzung bezogen, umfasst;
d. die Kohlehydrate 5 bis 50 Gew.-% Ballaststoffe, auf das Gesamtgewicht der Kohlehydrate in der Zusammensetzung bezogen, umfassen; und
wobei der Gewichtsprozentsatz an Aminosäuren mindestens 5 %, an Fett bis zu 20 % und an Kohlehydraten 10 bis 70 %, auf das Trockengewicht der Zusammensetzung bezogen, beträgt.

14. Zusammensetzung nach Anspruch 13, des Weiteren probiotische Bakterien umfassend.

## Revendications

1. Utilisation d'une formulation nutritionnelle administrable par voie orale comprenant (a) des acides aminés libres en tant qu'unique source de protéine, (b) une matière grasse et (c) des hydrates de carbone, dans laquelle a. la composition des acides aminés comprend au moins de la L-alanine, de la L-arginine, de l'acide L-aspartique, de la L-cystine, de la glycine, de la L-histidine, de la L-isoleucine, de la L-lysine, de la L-méthionine, de la L-phénylalanine, de la L-proline, de la L-sérine, de la L-thréonine, du L-tryptophane, de la L-tyrosine, de la L-valine, de la L-carnitine et de la taurine;
b. la matière grasse comprend des acides gras polyinsaturés à chaîne longue; et
c. les hydrates de carbone comprennent des fibres
pour la fabrication d'un médicament pour l'administration en tant qu'unique source quotidienne de protéine ou en tant que complément nutritionnel de régimes alimentaires d'exclusion, lesdits acides aminés dans la formulation nutritionnelle étant l'unique source quotidienne de protéine ou complétant en partie les déficiences provoquées par l'exclusion de certaines protéines dans l'alimentation pour le traitement de troubles envahissants du développement.

2. Utilisation selon la revendication 1, dans laquelle le trouble envahissant du développement est l'autisme.

3. Utilisation selon la revendication 1, dans laquelle le trouble envahissant du développement est un trouble d'Asperger ou un trouble de Rett.

4. Utilisation selon la revendication 1, dans laquelle
b. la matière grasse comprend au moins 10% en poids d'acides gras polyinsaturés à chaîne longue par rapport au poids de la matière grasse dans la composition;
c. les hydrates de carbone comprennent entre 5 et 50% en poids de fibres par rapport au poids total des hydrates de carbone dans la composition; et
dans laquelle le pourcentage en poids des acides aminés est d'au moins 5%, de la matière grasse jusqu'à 20% et des hydrates de carbone d'entre 10 et 70% du poids sec de la composition.

5. Utilisation selon la revendication 4, dans laquelle les acides aminés libres comprennent de plus de la L-glutamine.

6. Utilisation selon l'une quelconque des revendications 1 à 5, dans laquelle la formulation incorpore des bactéries probiotiques.

7. Formulation nutritionnelle comprenant (a) des acides aminés en tant qu'unique source de protéine, (b) une matière grasse et (c) des hydrates de carbone, dans laquelle
a. la composition des acides aminés comprend au moins de la L-alanine, de la L-arginine, de l'acide L-aspartique, de la L-cystine, de la glycine, de la L-histidine, de la L-isoleucine, de la L-lysine, de la L-méthionine, de la L-phénylalanine, de la L-proline, de la L-sérine, de la L-thréonine, du L-tryptophane, de la L-tyrosine, de la L-valine, de la L-carnitine et de la taurine;
b. la matière grasse comprend des acides gras polyinsaturés à chaîne longue; et
c. les hydrates de carbone comprennent des fibres
pour le traitement de troubles envahissants du développement.

8. Formulation selon la revendication 7, dans laquelle le trouble envahissant du développement est l'autisme.

9. Formulation selon la revendication 7, dans laquelle le trouble envahissant du développement est un trouble d'Asperger ou un trouble de Rett.

10. Formulation selon la revendication 7, dans laquelle
b. la matière grasse comprend au moins 10% en poids d'acides gras polyinsaturés à chaîne longue par rapport au poids de la matière grasse dans la composition;
c. les hydrates de carbone comprennent entre 5 et 50% en poids de fibres par rapport au poids total des hydrates de carbone dans la composition; et
dans laquelle le pourcentage en poids des acides aminés est d'au moins 5%, de la matière grasse jusqu'à 20% et des hydrates de carbone d'entre 10 et 70% du poids sec de la composition.

11. Formulation selon la revendication 10, dans laquelle les acides aminés libres comprennent de plus de la L-glutamine.

12. Formulation selon l'une quelconque des revendications 7 à 11, laquelle formulation incorpore des bactéries probiotiques.

13. Composition comprenant des acides aminés en tant qu'unique source de protéine, une matière grasse et des hydrates de carbone, dans laquelle
b. la composition des acides aminés comprend au moins de la L-alanine, de la L-arginine, de l'acide L-aspartique, de la L-cystine, de la glycine, de la L-histidine, de la L-isoleucine, de la L-lysine, de la L-méthionine, de la L-phénylalanine, de la L-proline, de la L-sérine, de la L-thréonine, du L-tryptophane, de la L-tyrosine, de la L-valine, de la L-carnitine et de la taurine;
c. la matière grasse comprend au moins 10% en poids d'acides gras polyinsaturés à chaîne longue par rapport au poids de la matière grasse dans la composition;
d. les hydrates de carbone comprennent entre 5 et 50% en poids de fibres par rapport au poids total des hydrates de carbone dans la composition; et
dans laquelle le pourcentage en poids des acides aminés est d'au moins 5%, de la matière grasse va jusqu'à 20% et des hydrates de carbone d'entre 10 et 70% du poids sec de la composition.

14. Composition selon la revendication 13, comprenant en outre des bactéries probiotiques.
